(19) Europäisches Patentamt
European Patent Office
Office européen des brevets

(11) Publication number: **0 054 390**
**B1**

(12) # EUROPEAN PATENT SPECIFICATION

(45) Date of publication of patent specification: **25.07.84**

(51) Int. Cl.³: **C 07 J 1/00**, C 07 J 5/00, C 07 J 7/00, C 07 J 71/00

(21) Application number: **81305748.6**

(22) Date of filing: **04.12.81**

(54) **Dehalogenation of steroids.**

(30) Priority: **05.12.80 US 213447**
**15.05.81 US 263833**

(43) Date of publication of application:
**23.06.82 Bulletin 82/25**

(45) Publication of the grant of the patent:
**25.07.84 Bulletin 84/30**

(84) Designated Contracting States:
**BE CH DE FR GB IT LI NL SE**

(56) References cited:
**US - A - 3 480 622**

**JOURNAL OF THE AMERICAN CHEMICAL SOCIETY, vol. 88, no. 13, July 5, 1966 WASHINGTON D.C. (US) D.H.R. BARTON et al.: "Radical mechanisms in chromous ion reductions. An improved synthesis of 11 Beta-hydroxy steroids" pages 3016-3021**

(73) Proprietor: **THE UPJOHN COMPANY**
**301 Henrietta Street**
**Kalamazoo, Michigan 49001 (US)**

(72) Inventor: **Heather, James Brian**
**2198 Lupine Road**
**Hercules California 94547 (US)**
Inventor: **White, David Raymond**
**1918 Greenlawn Avenue**
**Kalamazoo Michigan 49007 (US)**

(74) Representative: **Perry, Robert Edward et al,**
**GILL JENNINGS & EVERY 53-64 Chancery Lane**
**London WC2A 1HN (GB)**

(58) References cited:
**JOURNAL OF ORGANIC CHEMISTRY, vol. 44, no. 1, January 5, 1979 American Chemical Society WASHINGTON D.C. (US) H. PARNES et al.: "Simple method for the reductive dehalogenation of 9 alpha-bromo steroids" pages 151-152**

**EP 0 054 390 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for the dehalogenation, and specifically the dechlorination or debromination, of steroids.

A large number of commercially valuable pharmacologically active steroids have 11$\beta$-hydroxy-$\Delta^4$-3-keto or 11$\beta$-hydroxy-$\Delta^{1,4}$-3-keto functionality. These steroids include hydrocortisone, 11$\beta$-hydroxyprogesterone, prednisolone, 6$\alpha$-methylprednisolone and 16-methylprednisolone, and analogues thereof. In the process of introducing the 11$\beta$-hydroxyl group, a 9$\alpha$-halo (chlorine or bromine) atom is also sometimes introduced. It is therefore often desirable to use a process which dehalogenates a 9$\alpha$-chloro or 9$\alpha$-bromo-11$\beta$-hydroxy steroid to the corresponding 11$\beta$-hydroxy steroid. Many known processes produce too much of the 9(11)-unsaturated compound as a by-product, to be of value; see J. Am. Chem. Soc. *79,* 1130 (1957) which discusses the problems of trying to debrominate a 9$\alpha$-bromo-11$\beta$-hydroxy steroid to form the corresponding 11$\beta$-hydroxy steroid. Using chromous chloride and zinc in acetic acid, pure 9(11)-dehydro compound was obtained in about 80% yield.

A process capable of being developed commercially is disclosed in U.S. Patent Specification No. 3,480,622. This process involves reacting a 9$\alpha$-bromo-11$\beta$-hydroxy steroid with a salt of a polyvalent metal, in which the metal is capable of being oxidised to a higher valence state in the presence of a substance capable of providing hydrogen free radicals. Reducing agents disclosed include ferrous, titanous, thallous, stannous and chromous salts. The preferred chromous salts are the chromous salts of an organic carboxylic acid, preferably a $C_{1-6}$ alkanoic acid such as acetic, propionic or butyric acid. Chromous acetate is "especially suitable".

In U.S. Patent Specification No. 3,480,622, the substances disclosed as being capable of providing hydrogen radicals include $H_3PO_2$, hydrides such as triarylsilanes and triaryltin hydrides, 1,4-dihydroaromatic compounds such as 1,4-dihydrobenzene and 1-benzyl-1,4-dihydronicotinamide, related dienes such as cyclopentadiene and, in particular, thiols. The thiols are alleged to increase greatly the yield of the desired dehalogenated product. The thiols may be aliphatic, araliphatic or aromatic and can carry any substituents such as hydroxy, ether, thioether, keto, carboxyl and esterified carboxyl groups. Unsubstituted hydrocarbon groups are preferred, including lower alkyl groups and unsubstituted aryl groups such as phenyl. A mechanistic explanation for the process is given in Tetrahedron Letters *43,* 3151 (1964), and a more detailed account is found in J. Am. Chem. Soc. *88,* 3016 (1966).

According to the present invention, a process for the preparation of an 11$\beta$-hydroxy steroid comprises reacting a 9$\alpha$-bromo- or 9$\alpha$-chloro-11$\beta$-hydroxy steroid with chromous sulfate or chromous chloride or a mixture thereof, and $HS—CH_2—COOH$.

One example of the invention is the conversion shown in Chart A, and is a process for the preparation of an 11$\beta$-hydroxy pregnane (II), which comprises reacting a 9$\alpha$-halo-11$\beta$-hydroxy pregnane (I) with chromous chloride or chromous sulfate, or a mixture thereof, and $HS—CH_2—COOH$.

A second example of the invention is the conversion shown in Chart B, and is a process for the preparation of an 11$\beta$-hydroxy androstane (IV), which comprises reacting a 9$\alpha$-halo-11$\beta$-hydroxy androstane (III) with chromous chloride or chromous sulfate, or a mixture thereof, and $HS—CH_2—COOH$.

A third example of the invention is the conversion shown in Chart C, and is a process for the preparation of an 11$\beta$,17$\alpha$-dihydroxyprogesterone (VI), which comprises reacting a 9$\alpha$-halo-11$\beta$,17$\alpha$-dihydroxyprogesterone (V) with chromous chloride or chromous sulfate, or a mixture thereof, and $HS—CH_2—COOH$.

A fourth example of the invention is the conversion shown in Chart D, and is a process for the preparation of an 11$\beta$-hydroxyprogesterone (VIII), which comprises reacting a 9$\alpha$-halo-11$\beta$-hydroxyprogesterone (VII) with chromous chloride or chromous sulfate, or a mixture thereof, and $HS—CH_2—COOH$.

It has been discovered that chromous chloride, chromous sulfate, and mixtures thereof, can be preferable reducing agents. Chromous chloride and chromous sulfate have the advantage of ease of preparation, storage and handling. These reagents can be prepared in higher yield than chromous acetate. A slight excess of chromous sulfate over theory brings about rapid (5 to 15 minutes) and complete reduction of 9$\alpha$-bromo-11$\beta$-hydroxysteroids when used with the co-reductant thioglycolic acid.

It has been discovered that thioglycolic acid can increase yield over that which is expected; its use can make the work-up procedure simple. With respect to known thiols, the use of thioglycolic acid can permit (1) a more versatile choice of reactant medium, (2) faster reaction, (3) greater ease of product isolation and (4) higher yields of 11$\beta$-hydroxy steroid product (II or IV).

Virtually all 9$\alpha$-chloro- or 9$\alpha$-bromo-11$\beta$-hydroxy steroids can be used in the present process. The preferred 9$\alpha$-halo-11$\beta$-hydroxy starting material (I, III, V, or VII) are well known or can be readily prepared by well known means, from known starting materials. It is preferred that the starting material is a bromo-steroid.

Polar inert solvents such as DMF, THF, and methanol give high yields.

The reaction temperature is not critical. From −50 to 100°C is suitable, 20—50°C being preferred.

Upon completion of the reaction as monitored by TLC, the reaction mixture is diluted with a volume of water greater than the total volume of the reaction mixture, preferably 2—10 times the reaction mixture volume. After addition of the water, the reaction mixture is stirred and then filtered. The filter cake is washed and then processed in an appropriate manner, depending on the next chemical reaction it will undergo. The filtrate can be extracted with an organic solvent to obtain additional product.

## Definitions

The definitions and explanations below are for the terms as used throughout the specification and claims.

All temperatures are in degrees Centigrade

TLC refers to thin-layer chromatography.

GLC refers to gas-liquid chromatography.

THF refers to tetrahydrofuran.

DMF refers to dimethylformamide.

DMSO refers to dimethyl sulfoxide.

Saline refers to an aqueous saturated sodium chloride solution.

$R_6$ is a fluorine atom or methyl group.

$R_9$ is a chlorine or bromine atom.

$R_{16}$ is a hydrogen atom or $\alpha$- or $\beta$-methyl or $\alpha$-hydroxyl group, when $R_{16}$ is $\alpha$-hydroxyl and when $R_{17}$ is a hydrogen atom, $R_{16}$ and $R_{17}$ can be a 16,17-acetonide.

$R_{17}$ is a hydrogen atom or a group of the formula —$COR_{17_A}$ where $R_{17_A}$ is alkyl of 1 through 4 carbon atoms or phenyl, when $R_{17}$ is a hydrogen atom and when $R_{16}$ is $\alpha$-hydroxyl, $R_{16}$ and $R_{17}$ can be a 16,17-acetonide.

$R_{21}$ is a hydrogen atom or a group of the formula —$COR_{21_A}$ where $R_{21_A}$ is alkyl of 1 through 4 carbon atoms or phenyl.

~ indicates the attached group can be in either the $\alpha$ or $\beta$ configuration.

---- is a single or double bond.

## Examples

Without further elaboration, it is believed that one skilled in the art can, using the preceding description, practice the present invention to its fullest extent. The following preferred specific embodiments are, therefore, to be construed as merely illustrative, and not limiting of the preceding disclosure in any way whatsoever.

### Example 1

Hydrocortisone acetate (II)

A mixture of 9$\alpha$-bromohydrocortisone acetate (I, 6.90 g) and thioglycolic acid (5.5 ml) in DMF (31 ml) is warmed to 45° with stirring under nitrogen. Over a period of about 5 minutes, a chromous sulfate solution (1.4 M, 13 ml) is added. The resulting mixture is stirred for 10 minutes and then cooled in an ice-water bath. The mixture is diluted with water (200 ml) and stirred at 5° for 30 minutes. The mixture is then filtered and the filter cake washed several times with water. The product is air dried to give the title compound, one spot by TLC, (5.15 g 89.2% chemical yield), which is identical with an authentic sample. An additional 3% of the title compound is obtained by liquid-liquid extraction of the filtrate.

### Example 2

6$\alpha$-Fluoro-11$\beta$,16$\alpha$,17$\alpha$,21-tetrahydroxypregn-4-ene-3,20-dione-16,17-acetonide 21-acetate (II)

9$\alpha$ - Bromo - 6$\alpha$ - fluoro - 11$\beta$,16$\alpha$,17$\alpha$,21 - tetrahydroxypregn - 4 - ene - 3,20 - dione - 16,17 - acetonide 21-acetate (I, 10.8 g) and thioglycolic acid (8.06 ml) are dissolved in DMSO (200 ml) and the mixture under nitrogen is cooled to 15°. Chromous sulfate (0.91 N aqueous solution, 21 ml) is added over a 36-minute period at 15—16° followed by the addition of water (700 ml, precooled to 1°) over a 2-minute period. The mixture is stirred for 10 minutes and the slurry is then filtered. The solids are washed with water to give the title compound.

### Example 3

Prednisolone (II)

Following the general procedure of Example 1 and making non-critical variations but starting with either 9$\alpha$-chloro or 9$\alpha$-bromoprednisolone (I), the title compound is obtained.

### Example 4

6$\alpha$-Methylprednisolone (II)

Following the general procedure of Example 1 and making non-critical variations but starting with either 9$\alpha$-chloro or 9$\alpha$-bromo-6$\alpha$-methylprednisolone (I), the title compound is obtained.

Example 5

11β-Hydroxyandrost-4-ene-3,17-dione (IV)

A solution of 9α-bromo-11β-hydroxyandrost-4-ene-3,17-dione (III, 3.00 g) thioglycolic acid (3.0 ml) in DMF (17 ml) is stirred under nitrogen in a 45—50° water bath. A chromous sulfate solution (1.4 M, 7.0 ml is added dropwise over about 5 minutes. The mixture is allowed to cool, and the resulting slurry is diluted by slow addition of water (50 ml). The mixture is cooled to about 5° and filtered. The filter cake is washed with water and air dried to give the title compound (2.03 g, 85.3% chemical yield) which is identical with an authentic sample. The filtrate is extracted to give additional material (0.34 g, 14.3%).

Example 6

11β-Hydroxyandrost-4-ene-3,17-dione (IV)

A solution of 9α-bromo-11β-hydroxyandrost-4-ene-3,17-dione (III, 2.00 g), thioglycolic acid (2.0 ml) in THF (20 ml) and water (5 ml) is stirred under nitrogen at 20—25°. A chromous sulfate solution (1.4 M, 5.5 ml) is added and the two-phase system is stirred. After four hours, another 4.5 ml of chromous sulfate solution is added and the mixture stirred overnight. The mixture is diluted with water and extracted with ethyl acetate. The ethyl acetate extracts are washed with water, a diluted sodium hydroxide solution, water and saline. The organic phase is dried over sodium sulfate and concentrated to a solid. The solid is crystallized from benzene-hexane to give a first crop of crystals of the title compound (1.38 g, 83.5% chemical yield) which are measured to be 99% pure as measured by GLC and is identical to an authentic sample.

Example 7

11β-Hydroxyandrost-4-ene-3,17-dione (IV)

A mixture of 9α-bromo-11β-hydroxyandrost-4-ene-3,17-dione (III, 0.25 g), zinc dust (0.15 g), chromic sulfate hydrate (0.05 g), thioglycolic acid (1 ml) and DMF (20 ml) are stirred under nitrogen at 20—25° for 20 hours. The crude product is isolated as in Example 3 and is shown by GLC to be the title compound in 83% chemical yield.

Example 8

6α-Fluoro-16α-methylpregna-1,4-diene-3,20-dione (VIII)

Following the general procedure of Example 1 and making non-critical variations but starting with 9α-bromo-6α-fluoro-16α-methylpregna-1,4-diene-3,20-dione (VII) the title compound is obtained.

CHART A

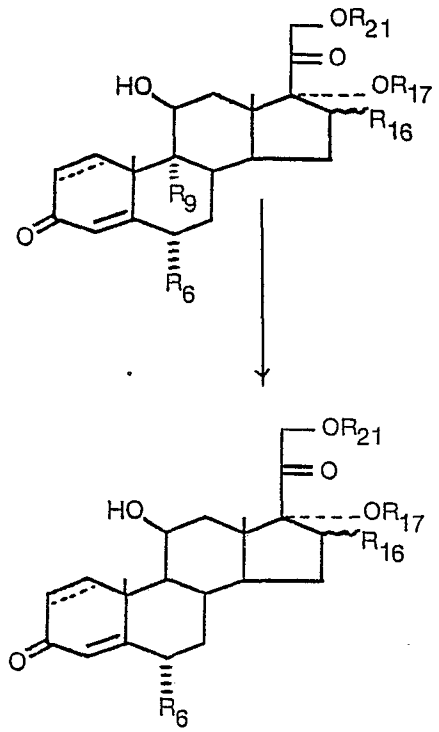

(I)

(II)

CHART B

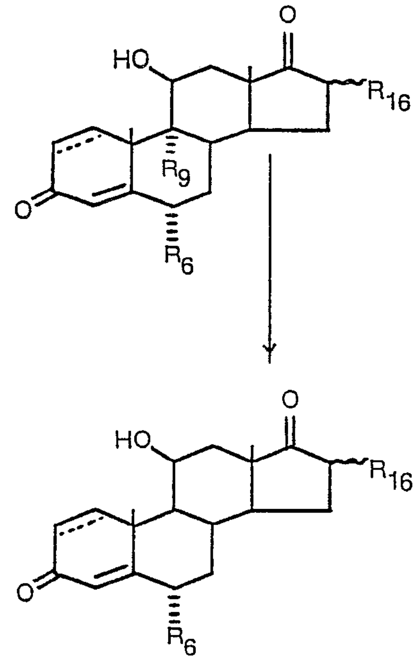

(III)

(IV)

CHART C

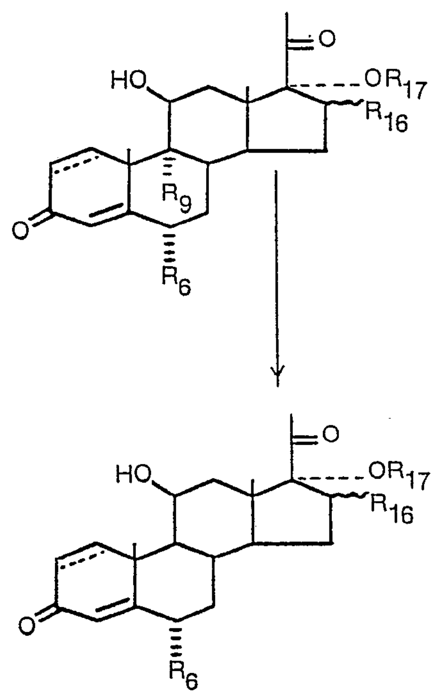

(V)

(VI)

CHART D

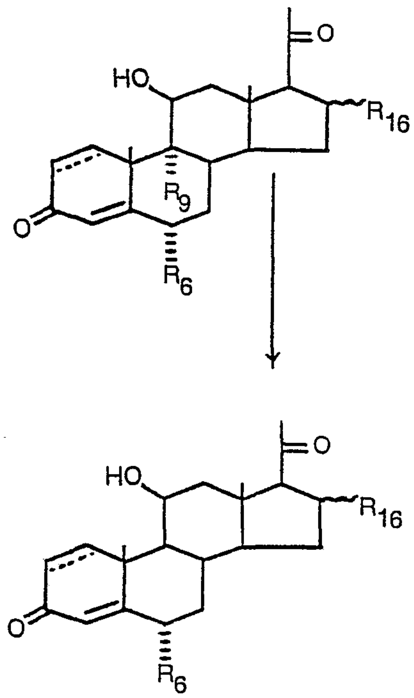

(VII)

(VIII)

**Claims**

1. A process for the preparation of an 11β-hydroxy steroid which comprises reacting a 9α-bromo-11β-hydroxy or a 9α-chloro-11β-hydroxy steroid with chromous sulfate or chromous chloride or mixtures thereof and $HS—CH_2—COOH$.

2. A process for the preparation of an 11β-hydroxy pregnane of the formula

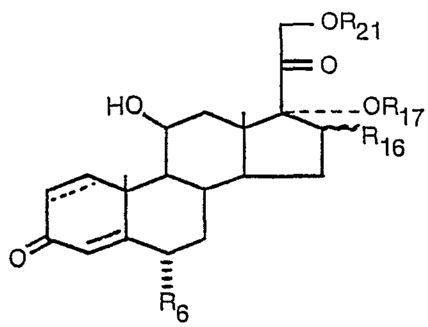

(II)

which comprises reacting a 9α-halo-11β-hydroxy pregnane of the formula

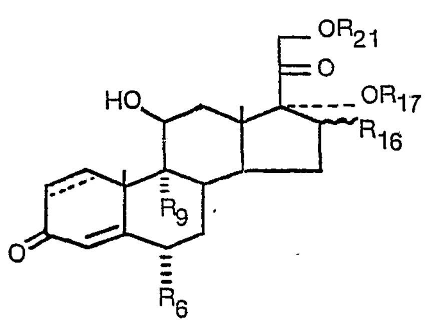

(I)

with chromous sulfate or chromous chloride or mixtures thereof and HS—$CH_2$—COOH, where $R_6$ is a fluorine atom or methyl group; $R_9$ is a chlorine or bromine atom; $R_{16}$ is a hydrogen atom or $\alpha$- or $\beta$-methyl or $\alpha$-hydroxyl group, when $R_{16}$ is $\alpha$-hydroxyl and when $R_{17}$ is a hydrogen atom, $R_{16}$ and $R_{17}$ can be a 16,17-acetonide; $R_{17}$ is a hydrogen atom or a group of the formula —$COR_{17A}$ where $R_{17A}$ is alkyl of 1 through 4 carbon atoms or phenyl, when $R_{17}$ is a hydrogen atom and when $R_{16}$ is $\alpha$-hydroxyl, $R_{16}$ and $R_{17}$ can be a 16,17-acetonide; $R_{21}$ is a hydrogen atom or a group of the formula —$COR_{21A}$ where $R_{21A}$ is alkyl of 1 through 4 carbon atoms or phenyl; ---- is a single or double bond; and ~ indicates the attached group can be in either the $\alpha$ or $\beta$ configuration.

3. A process for the preparation of an 11$\beta$-hydroxy androstane of the formula

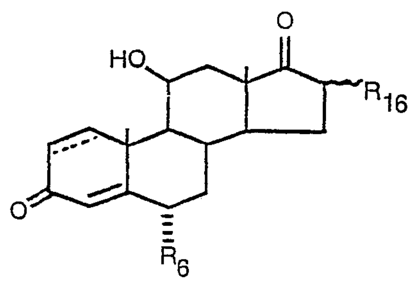

(IV)

which comprises reacting a 9$\alpha$-halo-11$\beta$-hydroxy androstane of the formula

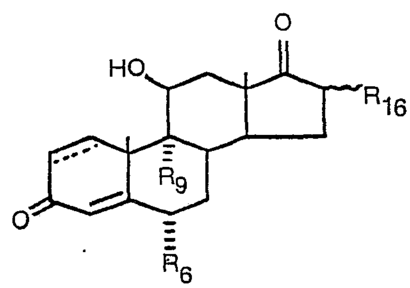

(III)

with chromous sulfate or chromous chloride or mixtures thereof and HS—$CH_2$—COOH, where $R_6$, $R_9$, $R_{16}$, ---- and ~ are defined in claim 2.

4. A process for the preparation of an 11$\beta$,17$\alpha$-dihydroxyprogesterone of the formula

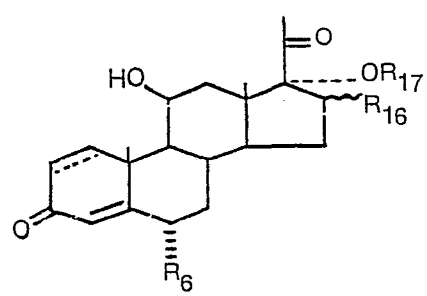

(VI)

which comprises reacting a 9$\alpha$-halo-11$\beta$,17$\alpha$-dihydroxyprogesterone of the formula

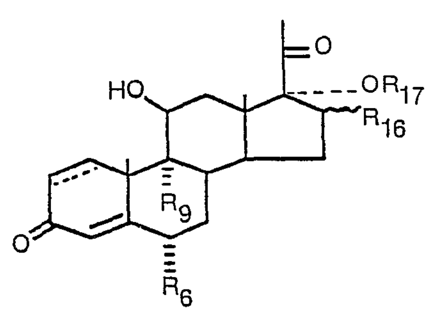

(V)

with chromous sulfate or chromous chloride or mixtures thereof and HS—$CH_2$—COOH, where $R_6$, $R_9$, $R_{16}$, $R_{17}$, .... and ~ are defined in claim 2.

5. A process for preparing an 11β-hydroxyprogesterone of the formula

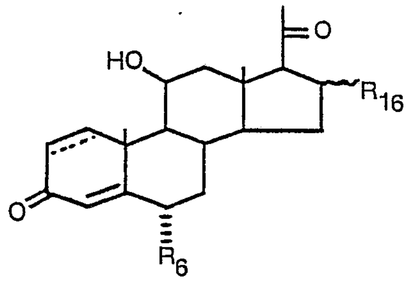

(VIII)

which comprises reacting a 9α-halo-11β-hydroxyprogesterone of the formula

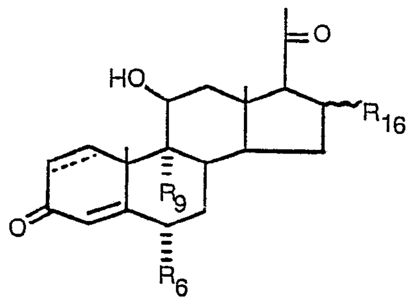

(VII)

wherein $R_6$, $R_9$, $R_{16}$, $R_{17}$, .... and ~ are defined in claim 2, with chromous sulfate or chromous chloride, or a mixture thereof, and HS—$CH_2$—COOH.

6. A process according to claim 2, wherein the steroid (II) is hydrocortisone acetate, prednisolone or 6α-methylprednisolone.

7. A process according to claim 3, wherein the androstane (IV) is 11β-hydroxyandrost-4-ene-3,17-dione.

8. A process according to claim 5, wherein the 11β-hydroxyprogesterone (VIII) is 6α-fluoro-16α-methylpregna-2,4-diene-3,20-dione.

9. A process according to any preceding claim, wherein the starting material is a 9α-bromo-11β-hydroxy steroid.

**Revendications**

1. Procédé de préparation d'un 11β-hydroxystéroïde, qui consiste à faire réagir un 9α-bromo-11β-hydroxy- ou un 9α-chloro-11β-hydroxystéroïde avec le sulfate chromeux ou le chlorure chomeux ou leurs mélanges et HS—$CH_2$—COOH.

2. Procédé de préparation d'un 11β-hydroxyprégnane de formule

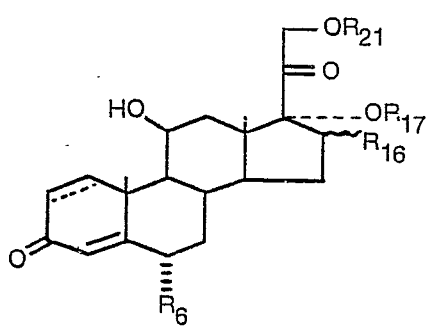

(II)

qui consiste à faire réagir un 9α-halogéno-11β-hydroxyprégnane de formule

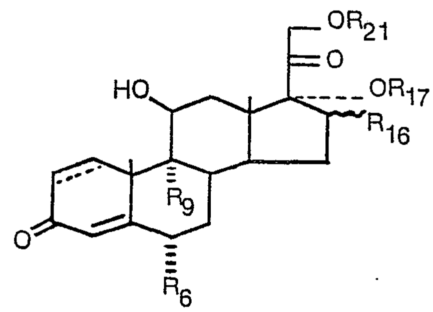

(I)

avec le sulfate chromeux ou le chlorure chromeux ou leurs mélanges et HS—$CH_2$—COOH, formules dans lesquelles $R_6$ est un atome de fluor ou un groupe méthyle; $R_9$ est un atome de chlore ou de brome; $R_{16}$ est un atome d'hydrogène ou un groupe $\alpha$- ou $\beta$-méthyle ou $\alpha$-hydroxyle, lorsque $R_{16}$ est un groupe $\alpha$-hydroxyle et lorsque $R_{17}$ est un atome d'hydrogène, $R_{16}$ et $R_{17}$ peuvent être un 16,17-acétonide; $R_{17}$ est un atome d'hydrogène ou un groupe de formule —$COR_{17_A}$ dans laquelle $R_{17_A}$ est un groupe alkyle ayant 1 à 4 atomes de carbone ou phényle, lorsque $R_{17}$ est un atome d'hydrogène et lorsque $R_{16}$ est un groupe $\alpha$-hydroxyle, $R_{16}$ et $R_{17}$ peuvent être un 16,17-acétonide; $R_{21}$ est un atome d'hydrogène ou un groupe de formule —$COR_{21_A}$ dans laquelle $R_{21_A}$ est un groupe alkyle ayant 1 à 4 atomes de carbone ou phenyle; ---- est une liaison simple ou double; et le signe ~ signifie que le groupe lié peut être en configuration $\alpha$ ou $\beta$.

3. Procédé de préparation d'un 11$\beta$-hydroxyandrostane de formule

(IV,

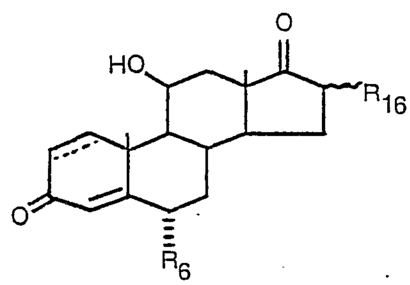

(IV)

qui consiste à faire réagir un 9$\alpha$-halogéno-11$\beta$-hydroxyandrostane de formule

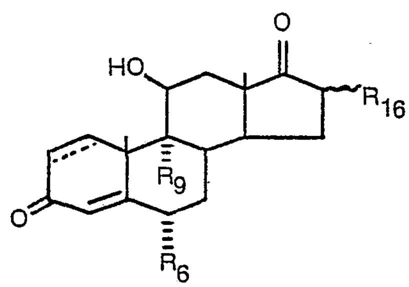

(III)

avec le sulfate chromeux ou le chlorure chromeux ou leurs mélanges et HS—$CH_2$—COOH, $R_6$, $R_9$, $R_{16}$, ---- et le signe ~ ayant les définitions données dans la revendication 2.

4. Procédé de préparation d'une 11$\beta$,17$\alpha$-dihydroxyprogestérone de formule

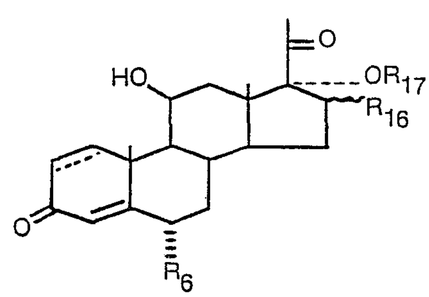

(VI)

qui consiste à faire réagir une 9α-halogéno-11β,17α-dihydroxyprogestérone de formule

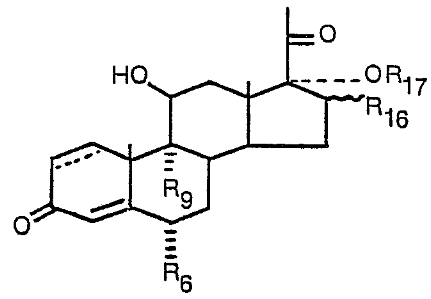

(V)

5. Procédé de préparation d'une 11β-hydroxyprogestérone de formule

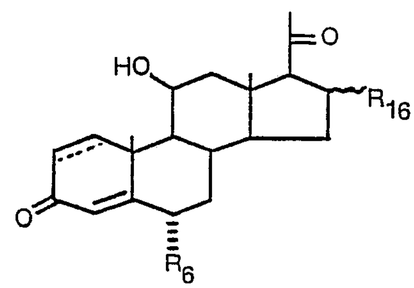

(VIII)

qui consiste à faire réagir un 9α-halogéno-11β-hydroxyandrostane de formule

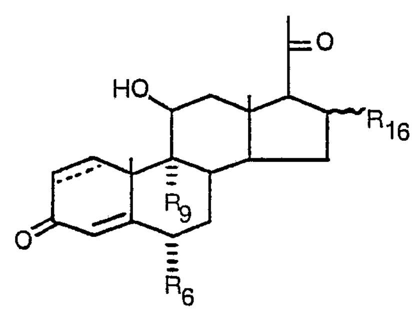

(VII)

dans laquelle $R_6$, $R_9$, $R_{16}$, $R_{17}$, .... et le signe ~ ayant les définitions données dans la revendication 2, avec du sulfate chromeux ou du chlorure chromeux ou leurs mélanges, et HS—$CH_2$—COOH.

6. Procédé suivant la revendication 2, dans lequel le stéroïde (II) est l'acétate d'hydrocortisone, la prednisolone ou la 6α-méthylprednisolone.

7. Procédé suivant la revendication 3, dans lequel l'androstane (IV) est la 11β-hydroxyandrost-4-ène-3,17-dione.

8. Procédé suivant la revendication 5, dans lequel la 11β-hydroxyprogestérone (VIII) est la 6α-fluoro-16α-méthylprégna-2,4-diène-3,20-dione.

9. Procédé suivant l'une quelconque des revendications précédentes, dans lequel la matière de départ est un 9α-bromo-11β-hydroxystéroïde.

**Patentansprüche**

1. Verfahren zum Herstellen eines 11β-Hydroxy-Steroids, bei welchem ein 9α-Bromo-11β-Hydroxy- oder ein 9α-Chloro-11β-Hydroxy-Steroid mit chromhaltigem Sulfat oder chromhaltigem Chlorid oder Gemischen derselben und mit HS—$CH_2$—COOH zur Reaktion gebracht wird.

2. Verfahren zum Herstellen eines 11$\beta$-Hydroxy-Pregnans mit der Formel

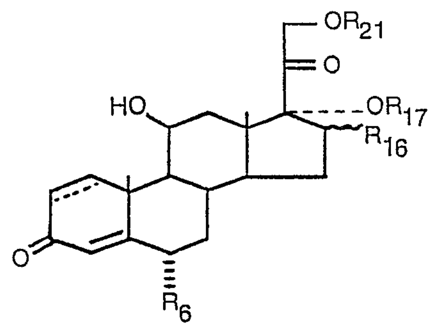

(II)

bei welchem ein 9$\alpha$-Halo-11$\beta$-Hydroxy-Pregnan mit der Formel

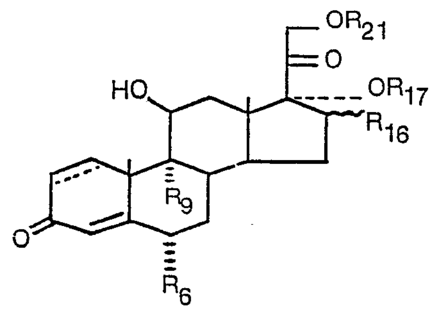

(I)

mit chromhaltigem Sulfat oder chromhaltigem Chlorid oder Gemischen derselben und mit HS—$CH_2$—COOH zur Reaktion gebracht wird, wobei $R_6$ ein Fluoratom oder eine Methylgruppe ist; $R_9$ ein Chlor- oder Bromatom ist; $R_{16}$ ein Wasserstoffatom oder $\alpha$- oder $\beta$-Methyl oder ein $\alpha$-Hydroxylgruppe ist und wenn $R_{16}$ $\alpha$-Hydroxyl und wenn $R_{17}$ ein Wasserstoffatom ist, kann $R_{16}$ und $R^{17}$ ein 16,17-Acetonid sein; $R_{17}$ ist ein Wasserstoffatom oder eine Gruppe mit der Formel —$COR_{17_A}$, wobei $R_{17_A}$ ein Alkyl mit 1 bis 4 Kohlenstoffatomen oder Phenyl ist, wenn $R_{17}$ ein Wasserstoffatom und $R_{16}$ ein $\alpha$-Hydroxyl ist, kann $R_{16}$ und $R_{17}$ ein 16,17-Acetonid sein; $R_{21}$ ist ein Wasserstoffatom oder eine Gruppe mit der Formel —$COR_{21_A}$, wobei $R_{21_A}$ Alkyl mit 1 bis 4 Kohlenstoffatomen oder Phenyl ist; ---- ist eine Einzel- oder Doppelbindung; und ~ bedeutet, daß die angefühgte Gruppe entweder in der $\alpha$- oder $\beta$-Anordnung vorliegen kann.

3. Verfahren zum Herstellen eines 11$\beta$-Hydroxy-Androstans mit der Formel

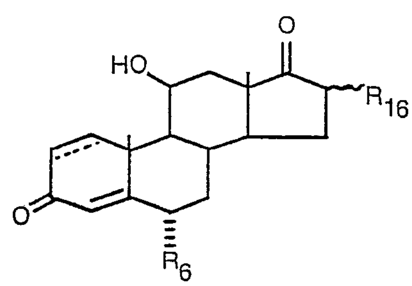

(IV)

bei welchem ein 9$\alpha$-Halo-11$\beta$-Hydroxy-Androstan mit der Formel

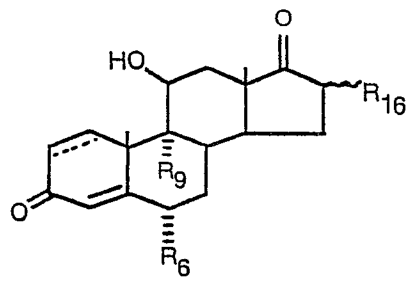

(III)

mit chromhaltigem Sulfat oder chromhaltigem Chlorid oder Gemischen derselben und mit HS—$CH_2$—COOH zur Reaktion gebracht wird, wobei $R_6$, $R_9$, $R_{16}$, ---- and ~ wie in Anspruch 2 definiert sind.

4. Verfahren zum Herstellen eines 11$\beta$,17$\alpha$-Dihydroxyprogesterons mit der Formel

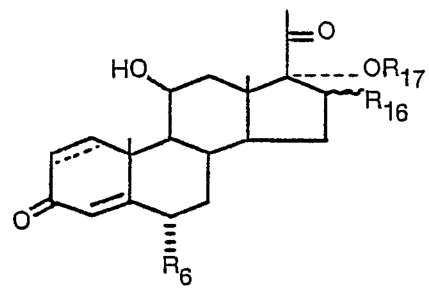

(VI)

bei welchem ein 9$\alpha$-Halo-11$\beta$,17$\alpha$-Dihydroxyprogesteron mit der Formel

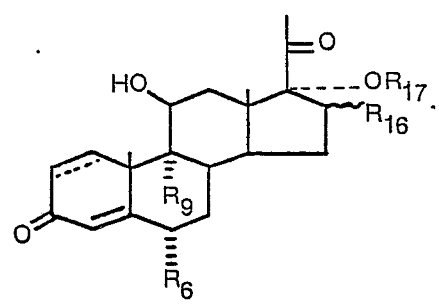

(V)

mit chromhaltigem Sulfat oder chromhaltigem Chlorid oder Gemischen derselben und mit HS—$CH_2$—COOH in Reaktion gebracht wird, wobei $R_6$, $R_9$, $R_{16}$, $R_{17}$, .... und ~ wie in Anspruch 2 definiert sind.

5. Verfahren zum Herstellen von 11$\beta$-Hydroxyprogesteron mit der Formel

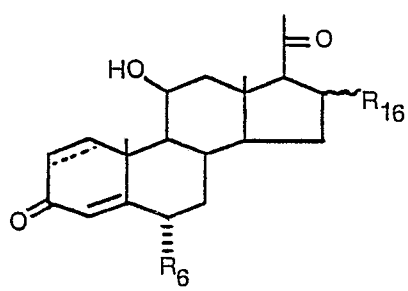

(VIII)

bei welchem ein 9$\alpha$-Halo-11$\beta$-Hydroxyprogesteron mit der Formel

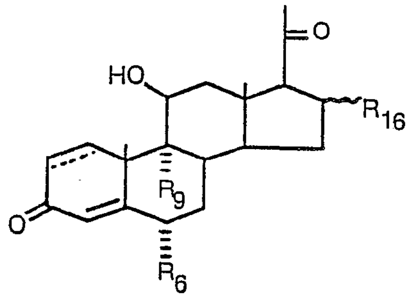

(VII)

( VII )

bei der $R_6$, $R_9$, $R_{16}$, $R_{17}$, .... und ~ wie in Anspruch 2 definiert sind, mit chromhaltigem Sulfat oder chromhaltigem Chlorid oder einem Gemisch derselben und mit HS—$CH_2$—COOH in Reaktion gebracht werden.

6. Verfahren nach Anspruch 2, bei dem das Steroid (II) Hydrocortison-Acetat, Prednisolon oder 6α-Methylprednisolon ist.

7. Verfahren nach Anspruch 3, bei dem das Androstan (IV) 11β-Hydroxyandrost-4-ol-3,17-dion ist.

8. Verfahren nach Anspruch 5, bei dem das 11β-Hydroxyprogesteron (VIII) 6α-Fluoro-16α-Methylpregna-2,4-diol-3,20-dion ist.

9. Verfahren nach einem der vorhergehenden Ansprüche, bei dem das Ausgangsmatrial ein 9α-Bromo-11β-Hydroxy-Steroid ist.